# EUROPÄISCHE PATENTSCHRIFT

(11) **EP 2 734 250 B1**
(45) Veröffentlichungstag und Bekanntmachung des Hinweises auf die Patenterteilung: **12.10.2016**
(21) Anmeldenummer: 12737700.0
(22) Anmeldetag: 12.07.2012
(51) Int. Cl.: A61M 1/06

(54) **BRUSTHAUBENEINHEIT**
BREASTSHIELD UNIT
ENSEMBLE TÉTERELLE DE TIRE-LAIT

(30) Priorität: 18.07.2011 CH 12012011
(43) Veröffentlichungstag der Anmeldung: 28.05.2014
(73) Patentinhaber: Medela Holding AG, 6340 Baar (CH)
(72) Erfinder: KHALIL, Gamal, CH-6340 baar (CH); FISCHER, René, CH-8057 Zürich (CH); SCHLIENGER, André, CH-8933 Maschwanden (CH)
(74) Vertreter: Clerc, Natalia
(86) Internationale Anmeldenummer: PCT/CH2012/000164
(87) Internationale Veröffentlichungsnummer: WO 2013/010286

(56) Entgegenhaltungen:
- EP-A1- 2 138 197
- US-A1- 2008 262 420
- US-A1- 2011 071 466
- US-B1- 6 461 324

## Beschreibung

### TECHNISCHES GEBIET

Die vorliegende Erfindung betrifft eine Brusthaubeneinheit gemäss Oberbegriff des Patentanspruchs 1 sowie eine Brustpumpe gemäss Oberbegriff des Patentanspruchs 13.

### STAND DER TECHNIK

Vorrichtungen zum Abpumpen von menschlicher Muttermilch sind hinlänglich bekannt. Manuell oder elektrisch betriebene Vakuumpumpen sind direkt oder über Vakuumleitungen mit einer Brusthaube verbunden, welche zur Aufnahme eines Teils der Mutterbrust inklusive der Brustwarze dient.

WO 2008/057218 offenbart eine Brustpumpe mit einer Vakuumpumpe, einer Vakuumleitung, einer Basis und einer an dieser Basis befestigbaren trichterförmigen steifen Brusthaube. Die Vakuumleitung verbindet die Vakuumpumpe mit der Basis. Um die Vakuumleitung und die Vakuumpumpe vor einer Verunreinigung zu schützen, ist in der Basis eine Barriere in Form einer grossflächigen Membran angeordnet. Diese Membran ist in einer Pumpkammer angeordnet, in welcher der von der Pumpeinheit erzeugte Unterdruck auf die andere Seite der Membran übermittelt wird.

WO 2011/037841 beschreibt ebenfalls eine Brustpumpe mit einem Basisteil, an welches eine trichterförmige steife Brusthaube, eine Vakuumleitung zur Verbindung mit der Vakuumpumpe und ein Milchsammelbehälter befestigbar sind. Das Basisteil weist zum Schutz der Vakuumleitung ebenfalls eine Membran auf.

US 7 223 255 und WO 2008/137678 zeigen sogenannte handfreie Brustpumpen, welche unter dem Büstenhalter getragen werden können. Das Brustinterface, welches dichtend auf die Mutterbrust angelegt wird, dient gleichzeitig als Membran der Vakuumpumpe. D.h. die Pumpkammer der Vakuumpumpe wird zwischen dem weichen Brustinterface und der Mutterbrust gebildet.

Eine weitere handfreie Brustpumpe ist in US 6 379 327 offenbart. Hier ist eine kleine Brusthaube im Büstenhalter angeordnet. Von dieser Brusthaube führen eine Vakuumleitung zu einer Vakuumpumpeneinheit und eine Milchleitung zu einem Milchsammelbehälter. Vakuumpumpeneinheit und Milchsammelbehälter sind in einem Gürtel, welchen die Mutter auf der Hüfte trägt, angeordnet.

In US 2008/0262420 ist an einer steifen Brusthaube ein flexibler Beutel zum Sammeln der Milch befestigt und beides im Büstenhalter getragen. Eine im oberen Bereich der Brusthaube mündende Vakuumleitung führt zu einer in einem Gürtel getragenen Vakuumpumpe.

Auch US 6 440 100 zeigt eine handfreie Brustpumpe. Auch hier führt eine Vakuumleitung von einer Brusthaube zu einer Vakuumpumpe. Diese Leitung wird gleichzeitig als Milchleitung verwendet.

US 2008/0039781 betrifft eine Tragvorrichtung für steife Brusthauben, wobei die Tragvorrichtung am Büstenhalter befestigt werden kann.

US 4 270 538 zeigt ein Brustschild, welches zum Auffangen von spontan ausfliessender Mutterinilch ausgebildet ist. Das Brustschild weist ein weiches Brustinterface und eine damit verbundene Kappe auf, in welche die Milch fliessen kann.

WO 2011/035448 offenbart eine Brustpumpe zum Abpumpen von menschlicher Muttermilch, bei welcher eine Vakuumleitung gleichzeitig als Milchleitung verwendet wird und eine Membran einer Membranvakuumpumpe als Medientrennung dient.

US 6 461 324 offenbart eine Brusthaube mit einem flexiblen Einsatz, einem Vakuumanschluss und einem Zwischenraum zwischen Brusthaube und Einsatz, in welchem ein Unterdruck erzeugt wird.

EP 2 138 197 beschreibt eine handbetätigbare Milchpumpe mit einer Membran.

### DARSTELLUNG DER ERFINDUNG

Es ist eine Aufgabe der Erfindung, eine verbesserte Vorrichtung zum Abpumpen von menschlicher Muttermilch zu schaffen.

Diese Aufgabe lösen eine Brusthaubeneinheit mit den Merkmalen des Patentanspruchs 1 sowie eine Brustpumpe mit den Merkmalen des Patentanspruchs 13.

Die erfindungsgemässe Brusthaubeneinheit zum Abpumpen von menschlicher Muttermilch weist ein Aufnahmeteil zur Aufnahme einer menschlichen Brustwarze, eine Unterdruckkammer zum Anlegen eines Unterdrucks an die Brustwarze, wobei das Aufnahmeteil in diese Unterdruckkammer mündet, sowie eine Membran zur Erzeugung eines Unterdrucks in der Unterdruckkammer auf.

Erfindungsgemäss ist die Membran ein- oder mehrstückig ausgebildet, und sie umgibt das Aufnahmeteil mindestens teilweise.

Alternativ oder zusätzlich ist die Membran, in Ausdehnungsrichtung einer Mittelachse des Aufnahmeteils betrachtet, auf einer der Brustwarze zugewandten Seite der Unterdruckkammer angeordnet. Diese Ortsangabe bedeutet nicht, dass die Membran die Unterdruckkammer von der Brustwarze trennt, sondern dass die Membran nicht wie im Stand der Technik bekannt, in Richtung der Mittelachse weiter entfernt von der Brustwarze angeordnet ist als die Unterdruckkammer. Diese Anordnung von Unterdruckkammer und Membran erlaubt die Ausbildung einer kompakten Brusthaubeneinheit.

Ebenfalls alternativ oder zusätzlich weist die erfindungsgemässe Brusthaubeneinheit einen Milchanschluss zur Verbindung der Unterdruckkammer mit einem Milchsammelbehälter auf. Die Membran zur Erzeugung eines Unterdrucks in der Unterdruckkammer ist, in Ausdehnungsrichtung einer Mittelachse des Aufnahmeteils betrachtet, auf einer der Mutterbrust zugewandten Seite dieses Milchanschlusses angeordnet. Auch hier bedeutet diese Ortsangabe wiederum das im obigen Abschnitt Gesagte. Derselbe Vorteil wie im vorherigen Beispiel wird hier ebenfalls erzielt.

Die erfindungsgemässe Brusthaubeneinheit lässt sich dank der speziellen Anordnung der Membran äusserst klein und kompakt ausbilden. Trotzdem lässt sich eine grossflächige Membran verwenden, so dass ein kleiner Hub der Membran genügt, um einen für das Abpumpen notwendigen Unterdruck im Bereich der Brusthaube zu erzeugen. Der Hubbedarf ist zudem verringert, weil das Totvolumen minimiert ist, d.h. die Unterdruckkammer im Bereich der Brustwarze ein kleines Volumen aufweist.

Der grosse Membrandurchmesser erleichtert zudem die Montage und auch die Reinigung der Vorrichtung. Die Medientrennung, d.h. die Trennung von Luft bzw. Vakuum und Milch, ist dank der grossen Fläche der Membran ebenfalls optimal gewährleistet.

Die Membran kann ein- oder mehrteilig ausgebildet sein. Sie enthält vorzugsweise eine kreis- oder teilkreisförmige Öffnung. Diese Öffnung ist vorzugsweise im Zentrum angeordnet. Sie ist in einer bevorzugten Ausführungsform vom Aufnahmeteil durchsetzt.

In einer bevorzugten Ausführungsform ist die Membran einstückig und ringförmig ausgebildet. Diese Membran hat den Vorteil, dass sie einfach herstellbar, einfach montierbar und einfach zu reinigen ist. Zudem weist sie eine grosse Fläche auf.

In einer anderen bevorzugten Ausführungsform besteht die Membran aus mindestens zwei Einzelteilen, vorzugsweise drei Einzelteilen. Diese Einzelteile umschliessen im montierten Zustand einen Kreis oder einen Teilkreis. Diese Ausführungsform hat den Vorteil, dass die übrige Gestaltung der Brusthaubeneinheit nicht an eine ringförmige Gestalt der Membran angepasst sein muss, sondern dass im Gegenteil die Membranteile der Form der Einheit angepasst werden können. Die Pumpkammer kann entsprechend der Teilung der Membran unterteilt sein, oder sie kann als durchgehende Pumpkammer ausgebildet sein. Die einzelnen Membranteile können identisch ausgebildet sein, oder sie können unterschiedliche Formen und Grössen aufweisen.

In einer anderen bevorzugten Ausführungsform ist nur ein einziges Membranteil vorhanden, welches auf einer Seite eine teilkreisförmige Einbuchtung aufweist. Diese Einbuchtung ist im montierten Zustand dem Aufnahmeteil zugeordnet und vorzugsweise vom Aufnahmeteil durchsetzt. Der dieser Einbuchtung entgegen gesetzte Rand der Membran ist vorzugsweise ebenfalls gebogen ausgebildet. Die Membran kann beispielsweise eine nierenähnliche Form aufweisen.

Die einstückige ringförmige Membran ist in ihrer Ruheposition, d.h. ohne angelegten Unterdruck, vorzugsweise kegelstumpfförmig ausgebildet, so dass sie der Form der Mutterbrust optimal angepasst ist. Die Teile der mehrstückigen Membran und die einstückige teilkreisförmige Membran sind vorzugsweise als Teile eines derartigen Kegelstumpfes ausgebildet. Die Membran ist aus einem weichen flexiblen Material gefertigt, insbesondere aus Silikon. Bevorzugt ist eine Härte von 60 Shore A.

Die Membran ist in einem Membrangehäuse bewegbar gehalten. Beispielsweise kann die Membran hierfür an ihrem Rand einen umlaufenden Wulst aufweisen, welcher in einer entsprechenden Aufnahme des Membrangehäuses gehalten ist.

Das Membrangehäuse ist zweiteilig mit einem brustseitigen und einem brustfernen Teil ausgebildet.

Die erfindungsgemässe Brusthaubeneinheit weist in einer bevorzugten Ausführungsform ein Brustinterface zur Anlage an eine Mutterbrust auf, welches an einer die Membran umschliessenden Gehäuseschale befestigbar ist. Die Gehäuseschale kann durch ein brustfernes Membrangehäuseteil oder durch ein dieses Membrangehäuseteil ebenfalls umschliessendes Teil gebildet sein. Das Brustinterface ersetzt vorzugsweise das üblicherweise verwendete Brustschild.

In einer bevorzugten Ausführungsform ist das Brustinterface aus einem weichen Material, insbesondere aus Silikon gefertigt. Bevorzugterweise weist es eine Shore A Härte von 50 auf.

In einer bevorzugten Ausführungsform weist das Brustinterface einen kegelstumpfförmigen oder zylindrischen ersten Stutzen zur Aufnahme der Brustwarze auf, wobei dieser erste Stutzen mit einem zweiten Stutzen der Unterdruckkammer verbindbar ist und wobei dieser erste Stutzen gemeinsam mit dem zweiten Stutzen das Aufnahmeteil bildet. Der kegelstumpfförmige Teil ist vorzugsweise sehr kurz, mit einem grossen Öffnungswinkel und grossflächig ausgebildet, so dass üblicherweise nur der Warzenhof und evtl. noch angrenzendes Gewebe von der Brusthaube kontaktiert werden.

Das Brustinterface kann ein von der Membran getrenntes Teil sein oder es kann einstückig mit dieser Membran verbunden sein.

Das Membrangehäuse ist vorzugsweise bewegbar relativ zur Gehäuseschale gehalten. Sie bildet vorzugsweise ein Membrangehänge mit einer darin angeordneten Membran, welche bewegbar innerhalb dieser Gehäuseschale angeordnet sind. Dies hat den Vorteil, dass das Membrangehäuse bei einer gebrauchsmässigen Verformung des Brustinterface nicht auch verformt wird und so die Pumpkammer und die Unterdruckkammer, d.h. das Totvolumen, in ihrem Volumen unverändert bleiben. Dies gewährleistet eine gleichförmige Pumpleistung im Gebrauch.

Die erfindungsgemässe Brusthaubeneinheit lässt sich als handfreie Einheit ausbilden und unter einem Büstenhalter tragen. In einer bevorzugten Ausführungsform ist ein Milchsammelbehälter in das Gehäuse der Einheit integriert oder an dieses unmittelbar ankoppelbar. Alternativ oder zusätzlich ist auch eine manuell betriebene oder eine motorbetriebene Vakuumpumpe im Gehäuse integriert oder-unmittelbar mit diesem verbunden. Bei Verwendung einer motorbetriebenen Vakuumpumpe ist vorzugsweise auch eine Stromversorgung im Gehäuse integriert oder mit diesem unmittelbar verbunden.

In einer anderen bevorzugten Ausführungsform ist die Brusthaubeneinheit über externe Leitungen mit einem Milchsammelbehälter und/oder mit einer externen Vakuumquelle verbunden.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. Gleiche Teile sind mit gleichen Bezugszeichen angegeben. In den Zeichnungen zeigen:
- Figur 1: eine schematische Darstellung einer Brustpumpe mit einer erfindungsgemässen Brusthaubeneinheit;
- Figur 2: die Brustpumpe gemäss Figur 1 in einer zweiten perspektivischen Ansicht;
- Figur 3: eine Explosionsdarstellung der Brusthaubeneinheit gemäss Figur 1;
- Figur 4: einen Längsschnitt durch die Brusthaubeneinheit gemäss Figur 1 bei Auflage auf einer Mutterbrust und bei offenem Milchanschluss;
- Figur 5: die Brusthaubeneinheit gemäss Figur 4 bei geschlossenem Milchanschluss;
- Figur 6: eine Explosionsdarstellung einer zweiten Ausführungsform der erfindungsgemässen Brusthaubeneinheit;
- Figur 7: eine Explosionsdarstellung einer dritten Ausführungsform der erfindungsgemässen Brusthaubeneinheit;
- Figur 8: die Brusthaubeneinheit gemäss Figur 7 im zusammengebauten Zustand;
- Figur 9: eine perspektivische Darstellung einer erfindungsgemässen Brustpumpe mit Brusthaubeineinheit, integriertem Milchsammelbehälter und integrierter Vakuumpumpe;
- Figur 10: die Brustpumpe gemäss Figur 9 ohne Deckel und
- Figur 11: eine Explosionsdarstellung der Brustpumpe gemäss Figur 9.

### BESCHREIBUNG BEVORZUGTER AUSFÜHRUNGSFORMEN

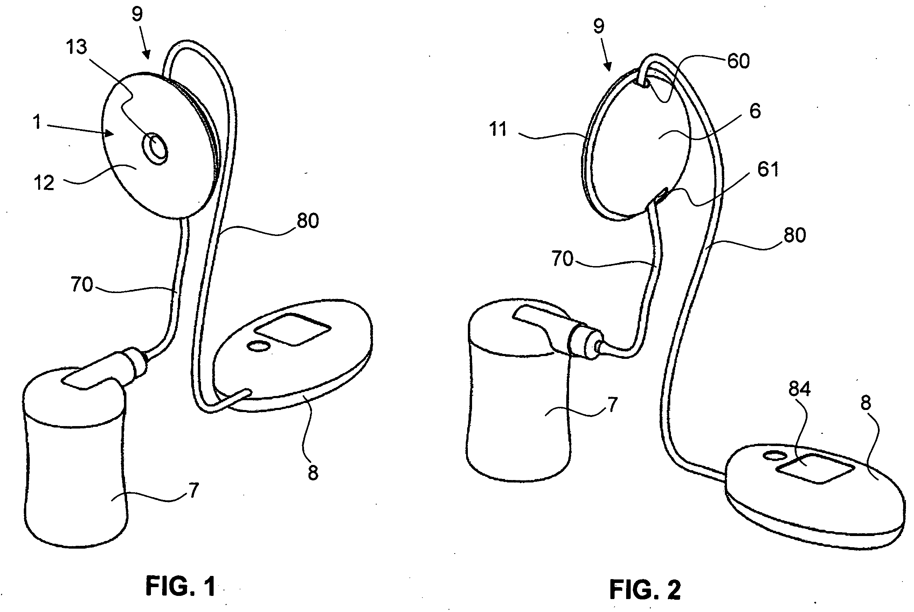

In den Figuren 1 bis 5 ist eine erste Ausführungsform einer erfindungsgemässen Brusthaubeneinheit 9 dargestellt.

Die Brusthaubeneinheit 9 weist ein Brustinterface 1 zur Auflage auf eine menschliche Mutterbrust und eine Schale 6 auf, an welcher das Brustinterface 1 lösbar befestigt ist. Das Brustinterface 1 ist vorzugsweise rund ausgebildet, wobei eine umlaufende Befestigungslippe 11 einen umlaufenden Befestigungsflansch 62 der Schale 6 umgibt.

Das Brustinterface 1 ist aus einem weichen elastischen Material, insbesondere aus Silikon, gefertigt und passt sich der Form der Mutterbrust an. Es weist eine Öffnung 13 auf, welche von einem kegelstumpfförmigen oder zylinderförmigen Stutzen 10 umschlossen ist. Diese Öffnung 13 ist vorzugsweise mittig innerhalb des Brustinterface 1 angeordnet.

Die Schale 6 ist vorzugsweise kalotten- oder halbkugelförmig ausgebildet. Sie ist vorzugsweise einstückig und starr. Üblicherweise ist sie aus einem Kunststoff gefertigt.

Im Ausführungsbeispiel gemäss den Figuren 1 bis 5 ist in der Schale 6 eine erste Durchgangsöffnung 60 vorhanden, durch welche eine Vakuumleitung 80, hier ein Silikonschlauch, mit einem entsprechenden Anschluss der Brusthaubeneinheit verbunden ist. Die Verbindung ist vorzugsweise lösbar ausgebildet. Diese Vakuumleitung 80 führt zu einer externen Saugpumpeneinheit 8, in welcher eine Vakuumpumpe angeordnet ist. Vorzugsweise handelt es sich um eine motorbetriebene Vakuumpumpe. Die Vakuumpumpe kann netzbetrieben sein. Zusätzlich oder alternativ kann in der Saupumpeneinheit eine Stromquelle integriert sein. Üblicherweise verfügt die Saugpumpeneinheit 8 über Bedienungselemente und einen Display 84.

Die Schale 6 ist mit einer zweiten Durchgangsöffnung 61 versehen. Durch diese ist ein erstes Ende einer Milchleitung 70, hier ebenfalls ein Silikonschlauch, mit einem Milchanschluss der Brustpumpeneinheit 9 lösbar verbindbar. Das andere Ende der Milchleitung 70 ist mit einem externen Milchsammelbehälter 7 lösbar verbunden.

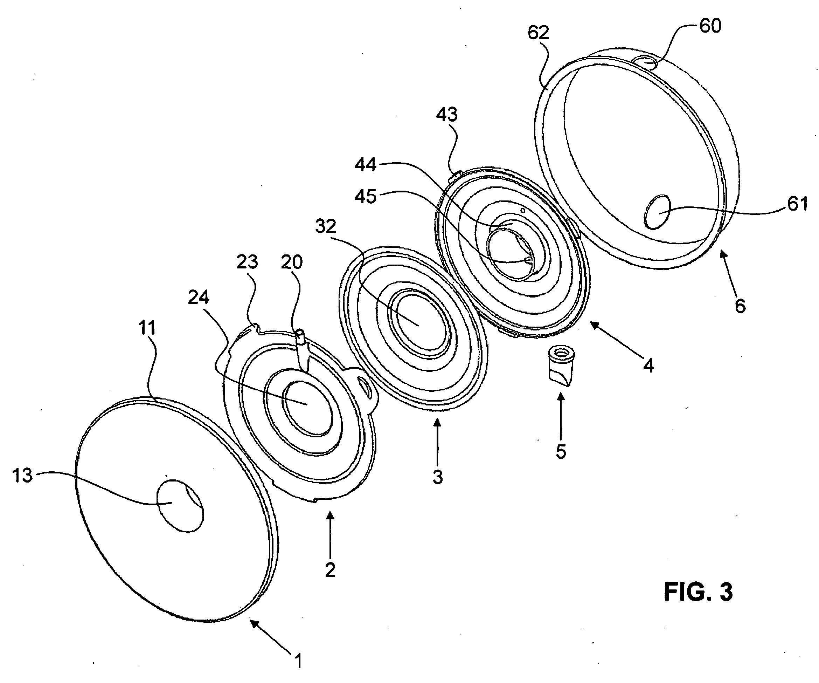

In Figur 3 sind die Einzelteile der erfindungsgemässen Brusthaubeneinheit 9 gut erkennbar. Das bereits beschriebene Brustinterface 1 bildet eine erste äussere Wand und die Schale 6 eine gegenüberliegende zweite äussere Wand der Einheit 9. Dazwischen ist ein brustseitiges Membrangehäuseteil 2, eine Membran 3, ein brustfernes Membrangehäuseteil 4 sowie ein Rückschlagventil 5, hier ein Entenschnabelventil, angeordnet.

Die zwei Membrangehäuseteile 2, 4 sind vorzugsweise aus einem steifen Material, insbesondere aus Kunststoff gefertigt. Sie sind vorzugsweise jeweils einstückig ausgebildet. Die Membran 3 besteht vorzugsweise aus einem flexiblen Material, insbesondere aus Silikon. Eine bevorzugte Härte beträgt 60 Shore A.

Die einzelnen Teile, mit Ausnahme des Rückschlagventils 5, weisen vorzugsweise eine runde Gestalt auf. Brustinterface 1, brustseitiges Membrangehäuseteil 2 und Membran 3 weisen vorzugsweise miteinander fluchtende, hier zentral angeordnete Öffnungen 13, 24, 32 auf. Das brustferne Membrangehäuseteil 4 weist einen mit diesen Öffnungen 13, 24, 32 fluchtenden Stutzen 44 auf.

Das brustnahe Membrangehäuseteil 2 weist an seinem äusseren Umfang mehrere, hier drei abgewinkelte Befestigungsösen 23 auf. Diese Befestigungsösen 23 lassen sich über entsprechende Befestigungsnasen 43 des brustfernen Membrangehäuseteils 4 stülpen. Dadurch wird in Kombination mit der eingelegten Membran 3 ein geschlossenes Membrangehäuse 2, 4 mit einer dichten ringförmigen Pumpkammer 46 ausgebildet. In dieser Pumpkammer 46 ist die Membran 3 bewegbar gehalten.

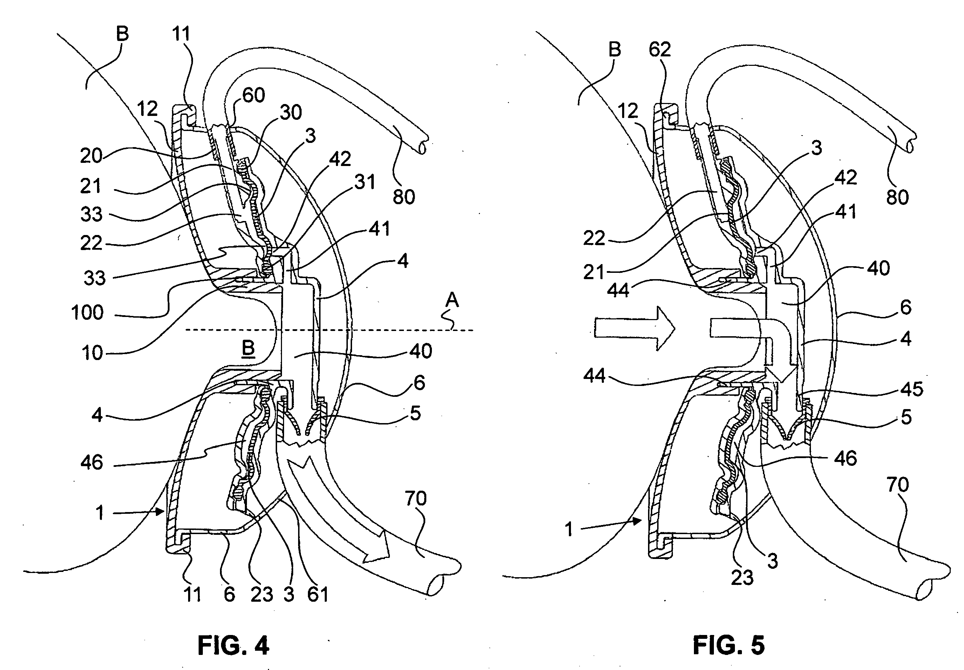

Dieser zusammengebaute Zustand ist in den Figuren 4 und 5 gut erkennbar. Die einstückige, ringförmige und vorzugsweise mit Sicken 33 versehene Membran 3 weist einen äusseren und einen inneren Befestigungswulst 30, 31 auf, welche in entsprechenden Aufnahmen gehalten, lagefixiert sind und eine dichtende Wirkung aufweisen. Diese Aufnahmen werden durch die zusammengefügten zwei Gehäuseteile 2, 4 gebildet. Die zwei Gehäuseteile 2, 4 bilden im Bereich der Membran 4 die ringförmige Pumpkammer 46 mit einer pumpenseitigen Öffnung 22 und einer brustseitigen Öffnung 42.

Die pumpenseitige Öffnung 22 führt über einen Vakuumkanal 21, welcher in einem Anschlussstutzen 20 endet, zur Vakuumleitung 80. Die brustseitige Öffnung 42 führt über einen Verbindungskanal 41 zu einer Unterdruckkammer 40. Diese Unterdruckkammer 40 ist durch das brustferne Membrangehäuseteil 4 begrenzt und zum Brustinterface 1 hin offen ausgebildet. Die Unterdruckkammer 40 weist einen Milchanschluss 45 auf, welcher vorzugsweise an dem dem Verbindungskanal 41 gegenüberliegenden Ende der Unterdruckkammer 40 angeordnet ist. In diesem Milchanschluss 45 ist das Rückschlagventil 5 angeordnet. Bei Überdruck in der Vakuumkammer 40 gegenüber Atmosphärendruck öffnet sich das Rückschlagventil 5 und erstellt eine Verbindung zur Milchleitung 70. Bei Unterdruck in der Vakuumkammer 40 schliesst das Rückschlagventil 5 und unterbricht so die Verbindung zur Milchleitung 70. Das Rückschlagventil 5 kann mit dem Milchanschluss 45 oder mit der Milchleitung 70 fest verbunden sein.

Die Vakuumkammer 40 geht in ihrem offenen Bereich in den Stutzen 44 über. Über diesen Stutzen 44 ist der Stutzen 10 des Brustinterface 1 gestülpt. Dieser Stutzen 10 weist hierfür eine ringförmige Aufnahmenut 100 auf.

Das Brustinterface 1 weist, wie hier erkennbar ist, vorzugsweise ein Basisteil 12 mit einer leicht trichterförmigen Gestalt und den daran einstückig angeformten Stutzen 10 auf. Dieser Stutzen definiert eine Mittelachse A. In den Figuren 4 und 5 ist das Brustinterface 1 auf eine menschliche Mutterbrust angelegt, so dass die Brustwarze B in das Aufnahmeteil, gebildet durch Stutzen 10 und Stutzen 44 hineinragt. Dadurch wird diese Öffnung der Unterdruckkammer 40 dichtend geschlossen.

Wird nun ein pulsierendes Vakuum bzw. ein Vakuum mit ändernder Frequenz und Intensität von der Saugpumpeinheit 8 generiert und über die Vakuumleitung 80 zur Pumpkammer 46 geleitet, so wird die Membran 3 zur pumpenseitigen Öffnung 22 hingezogen und verschliesst diese. Diese Situation ist in Figur 5 dargestellt. Durch die Bewegung der Membran 3 wird in der Pumpkammer 46 und somit in der Unterdruckkammer 40 ein Unterdruck erzeugt. Milch wird aus der Mutterbrust abgepumpt, wie dies die Pfeile in Figur 5 symbolisieren. Die Unterdruckkammer 40 füllt sich mindestens teilweise mit Muttermilch.

Wird nun der im Vakuumkanal 21 angelegte Unterdruck entsprechend dem von der Brustpumpe generierten Saugzyklus reduziert oder sogar auf Atmosphärendruck abgesenkt, so wird die Membran 3 zur brustseitigen Öffnung 42 hin gezogen, verschliesst diese und gibt dafür die pumpenseitige Öffnung 22 frei. Dadurch erhöht sich der Druck in der Unterdruckkammer 40 und das Rückschlagventil 5 öffnet sich. Die abgepumpte Milch kann durch die Milchleitung 70 abfliessen. Diese Situation ist in Figur 4 dargestellt.

Im Rhythmus des von der Saugpumpeneinheit 8 erzeugten Unterdrucks bewegt sich auch die Membran 3 und übermittelt so den Rhythmus und das Vakuumniveau an die Unterdruckkammer 40 weiter. Dank der relativ grossen Fläche der Membran 3 und des geringen Volumens der Unterdruckkammer 40 genügt ein relativ kleiner Hub, um einen zum Abpumpen notwendigen Unterdruck im Bereich der Brustwarze B zu erzeugen.

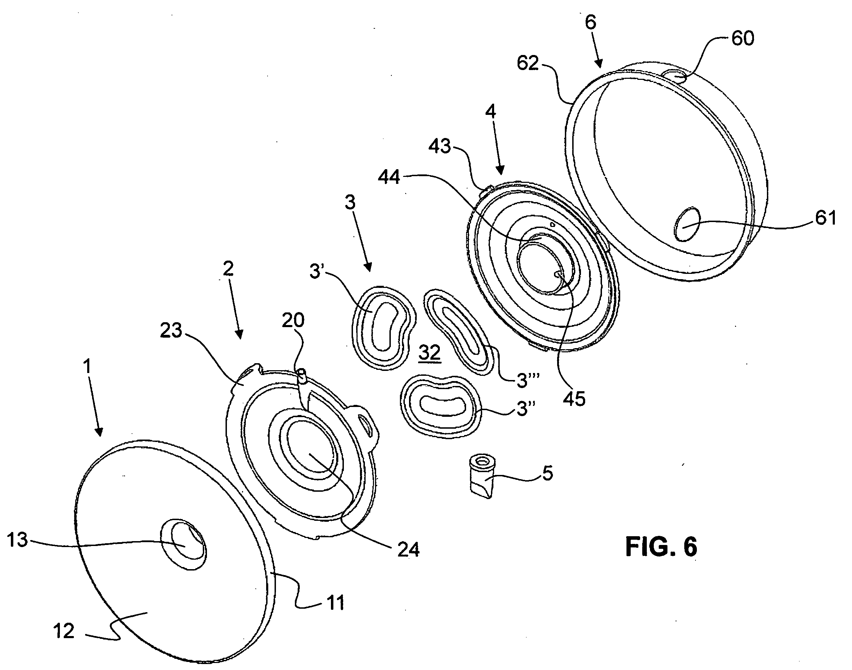

In Figur 6 ist ein zweites Ausführungsbeispiel dargestellt. Es weist im Wesentlichen dieselben Teile auf wie das erste Ausführungsbeispiel. Die Membran 3 ist jedoch mehrstückig ausgeführt. Hier sind drei Membranteile 3', 3", 3'" vorhanden, welche in entsprechenden Aufnahmen des Membrangehäuses 2, 4 gehalten sind und sich wiederum in einer Pumpkammer zwischen pumpenseitigen Öffnungen und brustseitigen Öffnungen hin und her bewegen können. Die Membranteile 3', 3", 3"' umschliessen eine kreisförmige Öffnung, welche wiederum vom Stutzen 10 des Brustinterface 1 und vom Stutzen 44 des brustfernen Membrangehäuseteils 4 durchsetzt ist.

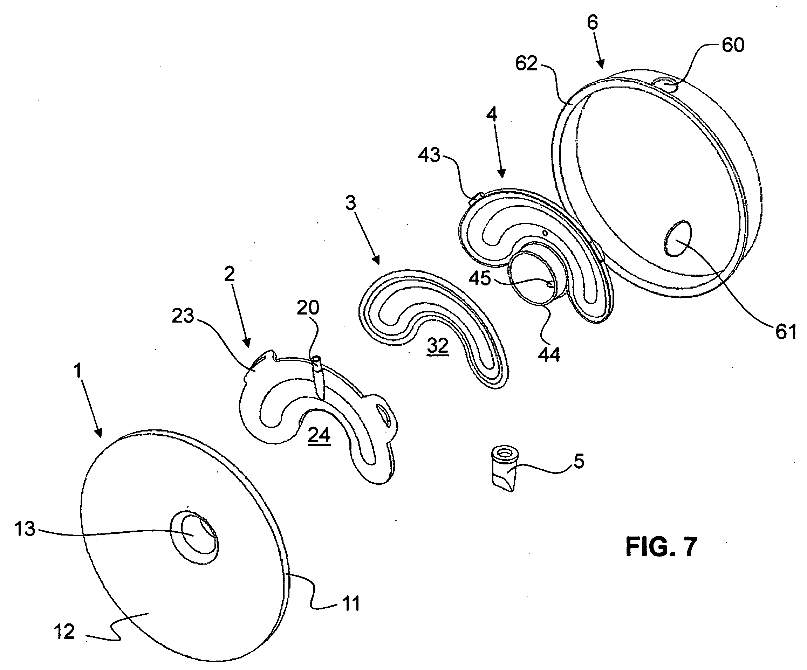

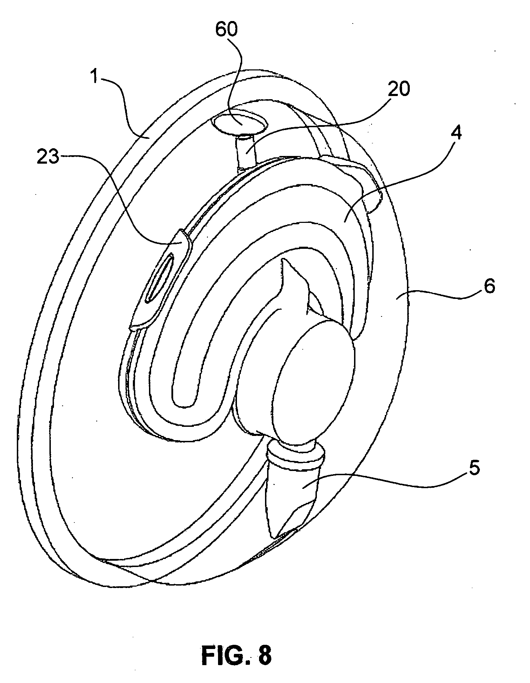

In den Figuren 7 und 8 ist ein drittes Ausführungsbeispiel dargestellt. Hier ist die Membran 3 wiederum einstückig ausgebildet, wobei sie lediglich einen Teilbereich eines Rings bildet. Sie kann auch nierenförmig ausgebildet sein. Sie umschliesst die zwei genannten Stutzen 10, 44 nur teilweise. Die Membrangehäuseteile 2, 4 weisen entsprechend ebenfalls nur einen teilkreisförmigen Querschnitt auf. Beim brustfernen Membrangehäuseteil 4 ist am Teilkreis der Stutzen 44 natürlich mit rundem Querschnitt und mit vollständig geschlossenem Mantel angeformt. In Figur 8 ist die Schale 6 durchsichtig dargestellt, damit das brustferne Membrangehäuseteil 4 gut erkennbar ist.

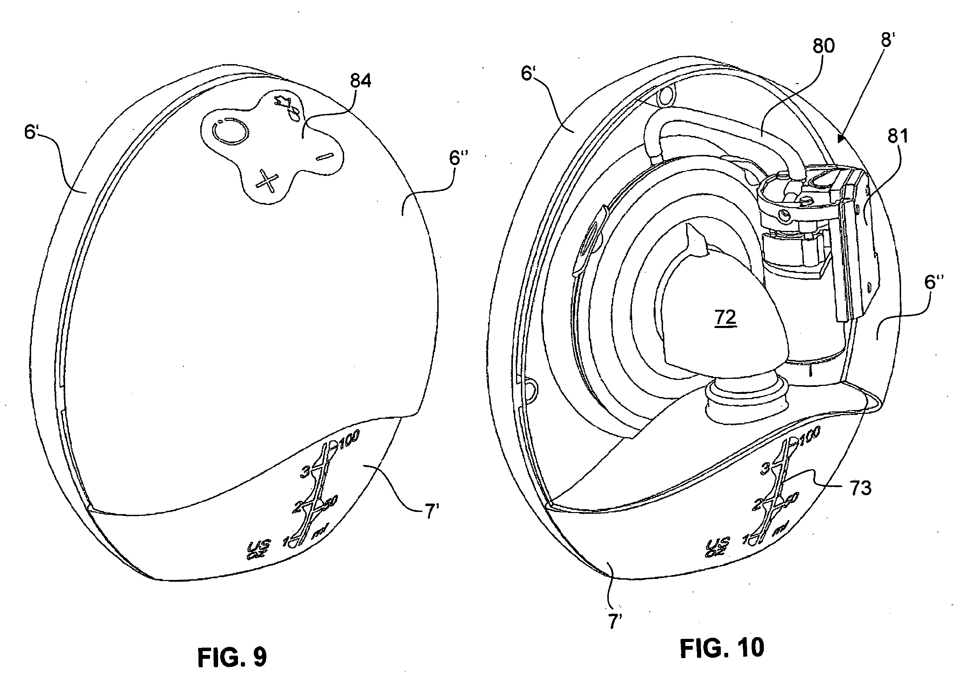

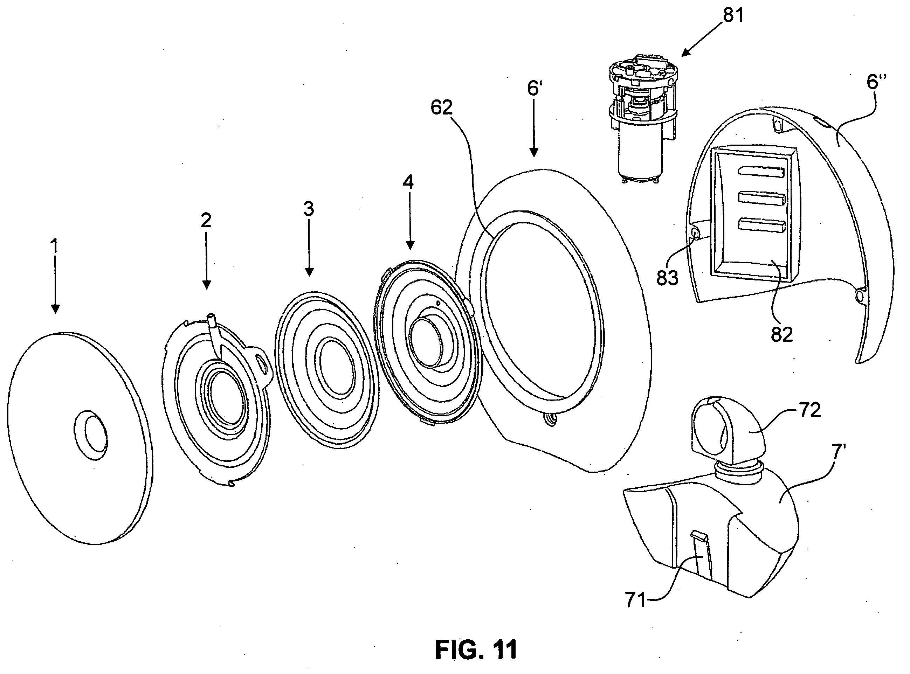

Die Figuren 9 bis 11 zeigen ein weiteres Ausführungsbeispiel, bei welchem die Brusthaubeneinheit als vollständige Brustpumpe mit einer integrierten Saugpumpeneinheit 8' ausgebildet ist. Das Brustinterface 1, die Membrangehäuseteile 2, 4 und die Membran 3 entsprechen der Ausführungsform gemäss den Figuren 1 bis 5. Es ist jedoch auch möglich, die Ausführungsformen gemäss den übrigen Figuren 6 bis 8 in einer derartigen Pumpe zu verwenden.

Anstelle der kalottenförmigen Schale 6 ist nun jedoch ein Schalenring 6' vorhanden. Dieser ist vorzugsweise wiederum starr ausgebildet und insbesondere aus Kunststoff gefertigt. Er weist eine Öffnung mit umlaufendem Befestigungsflansch 62 zur Befestigung des Brustinterface 1 auf. Seine Rückseite, d.h. seine brustferne Seite, ist teilweise durch einen Deckel 6" verschlossen. In diesem Deckel 6" ist eine Pumpenaufnahme 82 angeformt, in welcher eine Vakuumpumpe 81 gehalten ist. Vorzugsweise ist eine Stromquelle in dieser Vakuumpumpe 81 integriert. Die Vakuumpumpe 81 ist vorzugsweise eine Membranvakuumpumpe bekannter Art, welche mittels eines Elektromotors betrieben wird.

Auf der Aussenseite des Deckels 6" sind Bedienelemente 84 zur Betätigung der Vakuumpumpe 81 vorhanden. Die Brustpumpeneinheit umfasst ferner eine entsprechende Steuerung zur Übermittlung der benützerseitig über die Bedienelemente 84 eingegebenen Befehle an die Vakuumpumpe. Diese Steuerung kann separat im Gehäuse angeordnet sein oder in der Vakuumpumpe 81 integriert sein.

Im unteren Bereich, dem Deckel 6" anschliessend, ist ein Milchsammelbehälter 7' angeordnet. Er ist vorzugsweise wie der Deckel 6" aus Kunststoff gefertigt. An diesem Milchsammelbehälter 7' ist eine Einrastnase 71 angeformt, welche in eine entsprechende, in den Figuren nicht sichtbare Ausnehmung des Schalenrings 6' einrastbar ist. Dadurch ist der Milchsammelbehälter 7' lösbar mit dem Schalenring 6' verbunden. Der Milchsammelbehälter 7' weist zudem ein Kopplungsteil 72 mit einem integrierten, hier nicht dargestellten Ventil auf, welches an den Milchanschluss 45 des brustfernen Membrangehäuseteils 4 ansteckbar ist, so dass die abgepumpte Milch von der Unterdruckkammer 40 in den Innenraum des Milchsammelbehälters 7' fliessen kann. Das Ventil entspricht dem Rückschlagventil 5 der oben beschriebenen Ausführungsformen. Am Milchsammelbehälter 7' ist vorzugsweise eine Füllstandsanzeige 73 in Form einer Skala vorhanden. Entweder ist der Behälter 7' gesamthaft durchsichtig oder teiltransparent ausgebildet oder er weist im Bereich der Skala 73 ein durchsichtiges oder teiltransparentes Fenster auf.

Die erfindungsgemässe Brusthaubeneinheit ermöglicht eine Medientrennung im brustnahen Bereich. Sie ist klein und einfach zu reinigen und eignet sich insbesondere zur Verwendung als handfreie Brustpumpeneinheit, welche im Büstenhalter getragen werden kann.

**BEZUGSZEICHENLISTE**

| | | | |
|---|---|---|---|
| 1 | Brustinterface | 45 | Milchanschluss |
| 10 | Stutzen | 46 | Pumpkammer |
| 100 | Aufnahmenut | | |
| 11 | Befestigungslippe | 5 | Rückschlagventil |
| 12 | Basisteil | | |
| 13 | zentrale Öffnung | 6 | Schale |
| | | 6' | Schalenring |
| 2 | brustseitiges | 6" | Deckel |
| | Membrangehäuseteil | 60 | erste Durchgangsöffnung |
| 20 | Anschlussstutzen | 61 | zweite Durchgangsöffnung |
| 21 | Vakuumkanal | 62 | Befestigungsflansch |
| 22 | pumpenseitige Öffnung | | |
| 23 | Befestigungsöse | 7, 7' | Milchsammelbehälter |
| 24 | zentrale Öffnung | 70 | Milchleitung |
| | | 71 | Einrastnase |
| 3 | Membran | 72 | Kopplungsteil |
| 3', 3", 3"' | Membränteil | 73 | Füllstandsanzeige |
| 30 | äusserer Befestigungswulst | | |
| 31 | innerer Befestigungswulst | 8, 8' | Saugpumpeneinheit |
| 32 | zentrale Öffnung | 80 | Vakuumleitung |
| 33 | Sicke | 81 | Vakuumpumpe |
| | | 82 | Pumpenaufnahme |
| 4 | brustfernes | 83 | Schraubenloch |
| | Membrangehäuseteil | 84 | Bedienelement/Display |
| 40 | Unterdruckkammer | | |
| 41 | Verbindungskanal | 9 | Brusthaubeneinheit |
| 42 | brustseitige Öffnung | | |
| 43 | Befestigungsnase | A | Mittelachse |
| 44 | Stutzen | B | Brustwarze |

## Patentansprüche

1. Brusthaubeneinheit zum Abpumpen von menschlicher Muttermilch, wobei die Brusthaubeneinheit aufweist:
ein Aufnahmeteil (10) zur Aufnahme einer menschlichen Brustwarze;
eine Unterdruckkammer (40) zum Anlegen eines Unterdrucks an die Brustwarze,
wobei das Aufnahmeteil (10) in diese Unterdruckkammer (40) mündet und eine Membran (3) zur Erzeugung eines Unterdrucks in der Unterdruckkammer (40),
wobei die Membran (3) ein- oder mehrstückig ausgebildet ist, **dadurch gekennzeichnet, dass** sie das Aufnahmeteil (10) mindestens teilweise umgibt und dass die Brusthaubeneinheit ein brustseitiges Membrangehäuseteil (2) und ein brustfernes Membrangehäuseteil (4) aufweist, welche ein gemeinsames Membrangehäuse bilden, wobei die Membran (3) zwischen diesen zwei Membrangehäuseteile (2, 4) bewegbar gehalten ist.

2. Brusthaubeneinheit nach Anspruch 1, wobei das Aufnahmeteil eine Mittelachse (A) definiert und wobei in Ausdehnungsrichtung der Mittelachse (A) betrachtet die Membran (3) auf einer der Brustwarze zugewandten Seite der Unterdruckkammer (40) angeordnet ist.

3. Brusthaubeneinheit nach Anspruch 1, wobei das Aufnahmeteil eine Mittelachse (A) definiert und wobei in Ausdehnungsrichtung der Mittelachse (A) betrachtet die Membran (3) auf einer der Mutterbrust zugewandten Seite des Milchanschlusses (45) angeordnet ist.

4. Brusthaubeneinheit nach einem der Ansprüche 1 bis 3, wobei die Membran (3) eine kreis- oder teilkreisförmige Öffnung umgibt.

5. Brusthaubeneinheit nach Anspruch 4, **dadurch gekennzeichnet, dass** die Membran (3) einstückig und ringförmig ausgebildet ist.

6. Brusthaubeneinheit nach Anspruch 4, wobei die Membran (3) mindestens zwei Einzelteile (3', 3", 3'") aufweist, wobei die Einzelteile (3', 3", 3'") im montierten Zustand einen Kreis oder einen Teilkreis umschliessen.

7. Brusthaubeneinheit nach einem der Ansprüche 1 bis 6, wobei die Membran (3) in Ruheposition kegelstumpfförmig ausgebildet ist.

8. Brusthaubeneinheit nach einem der Ansprüche 1 bis 7, wobei sie ein Brustinterface (1) zur Anlage an eine Mutterbrust aufweist, welches an einer die Membran (3) umschliessenden Gehäuseschale (6) befestigbar ist.

9. Brusthaubeneinheit nach Anspruch 8, wobei das Brustinterface (1) einen kegelstumpfförmigen oder zylindrischen ersten Stutzen (10) zur Aufnahme der Brustwarze aufweist, wobei dieser erste Stutzen (10) mit einem zweiten Stutzen (44) der Unterdruckkammer (40) verbindbar ist und wobei dieser erste Stutzen (10) mit dem zweiten Stutzen (44) das Aufnahmeteil bilden.

10. Brusthaubeneinheit nach einem der Ansprüche 1 bis 9, wobei sie einen in die Brusthaubeneinheit integrierten Milchsammelbehälter (7') aufweist, wobei der Milchsammelbehälter (7') lösbar an der restlichen Brusthaubeneinheit angeordnet ist.

11. Brusthaubeneinheit nach einem der Ansprüche 1 bis 10, wobei sie einen mit einer Vakuumpumpe (8, 81) verbindbaren Vakuumanschluss (20) aufweist und wobei die Membran (3) den Vakuumanschluss (20) von der Unterdruckkammer (40) trennt.

12. Brusthaubeneinheit nach Anspruch 11, wobei die Membran (3) in einer Pumpkammer (46) angeordnet ist, welche einen ersten Ausgang (22) aufweist, der zum Vakuumanschluss (20) führt und einen zweiten Ausgang (42) aufweist, welcher zur Unterdruckkammer (40) führt.

13. Brustpumpe mit einer Brusthaubeneinheit gemäss einem der Ansprüche 11 oder 12, mit einer Vakuumleitung (80) und mit einer Vakuumpumpe (8, 81), wobei die Vakuumleitung (80) den Vakuumanschluss (20) mit der Vakuumpumpe (8, 81) verbindet.

14. Brustpumpe gemäss Anspruch 13, wobei die Brustpumpeneinheit eine die Membran (3) umschliessende Gehäuseschale (6) aufweist, in welcher die Vakuumpumpe (81) angeordnet ist.

## Claims

1. Breastshield unit for expressing human breastmilk, wherein the breastshield unit has:
a receiving part (10) for receiving a human nipple;
an underpressure chamber (40) for applying an underpressure to the nipple, wherein the receiving part (10) opens into this underpressure chamber (40), and
a membrane (3) for generating an underpressure in the underpressure chamber (40), wherein the membrane (3) is designed in one or more pieces,
**characterized in that** it at least partially surrounds the receiving part (10) and **in that** the breastshield unit has a breast-side membrane housing part (2) and a membrane housing part (4) facing away from the breast, which membrane housing parts (2, 4) form a common membrane housing, wherein the membrane (3) is held movably between these two membrane housing parts (2, 4).

2. Breastshield unit according to claim 1, wherein the receiving part defines a centre axis (A) and wherein, viewed in the direction of extension of the centre axis (A), the membrane (3) is arranged on a side of the underpressure chamber (40) facing towards the nipple.

3. Breastshield unit according to claim 1, wherein the receiving part defines a centre axis (A) and wherein, viewed in the direction of extension of the centre axis (A), the membrane (3) is arranged on a side of the milk port (45) facing towards the mother's breast.

4. Breastshield unit according to one of Claims 1 to 3, wherein the membrane (3) surrounds an opening in the shape of a circle or partial circle.

5. Breastshield unit according to Claim 4, **characterized in that** the membrane (3) is formed in one piece and has an annular shape.

6. Breastshield unit according to Claim 4, wherein the membrane (3) has at least two individual parts (3', 3'', 3"'), wherein the individual parts (3', 3", 3"') in the assembled state enclose a circle or a partial circle.

7. Breastshield unit according to one of Claims 1 to 6, wherein the membrane (3) has a frustoconical shape in the rest position.

8. Breastshield unit according to one of Claims 1 to 7, wherein it has a breast interface (1) for placing on a mother's breast, which breast interface (1) can be secured on a housing shell (6) enclosing the membrane (3).

9. Breastshield unit according to Claim 8, wherein the breast interface (1) has a frustoconical or cylindrical first stub (10) for receiving the nipple, wherein this first stub (10) can be connected to a second stub (44) of the underpressure chamber (40), and wherein this first stub (10) and the second stub (44) form the receiving part.

10. Breastshield unit according to one of Claims 1 to 9, wherein it has a milk collection container (7') integrated in the breastshield unit, wherein the milk collection container (7') is arranged releasably on the rest of the breastshield unit.

11. Breastshield unit according to one of Claims 1 to 10, wherein it has a vacuum port (20) that can be connected to a vacuum pump (8, 81), and wherein the membrane (3) separates the vacuum port (20) from the underpressure chamber (40).

12. Breastshield unit according to Claim 11, wherein the membrane (3) is arranged in a pump chamber (46) having a first outlet (22), which leads to the vacuum port (20), and a second outlet (42), which leads to the underpressure chamber (40).

13. Breastpump with a breastshield unit according to either of Claims 11 and 12, with a vacuum line (80) and with a vacuum pump (8, 81), wherein the vacuum line (80) connects the vacuum port (20) to the vacuum pump (8, 81).

14. Breastpump according to Claim 13, wherein the breastpump unit has a housing shell (6), which encloses the membrane (3) and in which the vacuum pump (81) is arranged.

## Revendications

1. Ensemble téterelle pour pomper du lait maternel humain, l'ensemble téterelle présentant :
une partie de réception (10) pour recevoir un mamelon humain ;
une chambre à dépression (40) pour l'application d'une dépression au mamelon, la partie de réception (10) débouchant dans cette chambre à dépression (40) et
une membrane (3) pour générer une dépression dans la chambre à dépression (40), la membrane (3) étant réalisée en une ou plusieurs parties,
**caractérisé en ce qu'**elle entoure au moins en partie la partie de réception (10) et **en ce que** l'ensemble téterelle présente une partie de boîtier de membrane du côté du sein (2) et une partie de boîtier de membrane du côté opposé au sein (4), qui forment un boîtier de membrane commun, la membrane (3) étant retenue de manière mobile entre ces deux parties de boîtier de membrane (2, 4).

2. Ensemble téterelle selon la revendication 1, dans lequel la partie de réception définit un axe médian (A) et dans lequel, vu dans la direction d'étendue de l'axe médian (A), la membrane (3) est disposée sur un côté de la chambre à dépression (40) tourné vers le mamelon.

3. Ensemble téterelle selon la revendication 1, dans lequel la partie de réception définit un axe médian (A) et dans lequel, vu dans la direction d'étendue de l'axe médian (A), la membrane (3) est disposée sur un côté du raccord à lait (45) tourné vers le sein maternel.

4. Ensemble téterelle selon l'une quelconque des revendications 1 à 3, dans lequel la membrane (3) entoure une ouverture en forme de cercle ou de cercle partiel.

5. Ensemble téterelle selon la revendication 4, **caractérisé en ce que** la membrane (3) est réalisée d'une seule pièce et sous forme annulaire.

6. Ensemble téterelle selon la revendication 4, dans lequel la membrane (3) présente au moins deux parties individuelles (3', 3'', 3"'), les parties individuelles (3', 3'', 3'''), dans l'état monté, englobant un cercle ou un cercle partiel.

7. Ensemble téterelle selon l'une quelconque des revendications 1 à 6, dans lequel la membrane (3) est réalisée sous forme tronconique dans la position de repos.

8. Ensemble téterelle selon l'une quelconque des revendications 1 à 7, dans lequel il présente une interface avec le sein (1) pour l'application contre un sein maternel, laquelle interface peut être fixée contre une coque de boîtier (6) entourant la membrane (3).

9. Ensemble téterelle selon la revendication 8, dans lequel l'interface avec le sein (1) présente un premier raccord (10) de forme tronconique ou cylindrique pour recevoir le mamelon, ce premier raccord (10) pouvant être connecté à un deuxième raccord (44) de la chambre à dépression (40) et ce premier raccord (10) formant avec le deuxième raccord (44) la partie de réception.

10. Ensemble téterelle selon l'une quelconque des revendications 1 à 9, présentant un récipient de collecte de lait (7') intégré à l'ensemble téterelle, le récipient de collecte de lait (7') étant disposé de manière détachable sur le reste de l'ensemble téterelle.

11. Ensemble téterelle selon l'une quelconque des revendications 1 à 10, présentant un raccord à vide (20) pouvant être connecté à une pompe à vide (8, 81) et dans lequel la membrane (3) sépare le raccord à vide (20) de la chambre à dépression (40).

12. Ensemble téterelle selon la revendication 11, dans lequel la membrane (3) est disposée dans une chambre de pompe (46) qui présente une première sortie (22) qui conduit au raccord à vide (20) et qui présente une deuxième sortie (42) qui conduit à la chambre à dépression (40).

13. Tire-lait comprenant un ensemble téterelle selon l'une quelconque des revendications 11 ou 12, comprenant une conduite à vide (80) et une pompe à vide (8, 81), la conduite à vide (80) reliant le raccord à vide (20) à la pompe à vide (8, 81).

14. Tire-lait selon la revendication 13, dans lequel l'ensemble téterelle présente une coque de boîtier (6) entourant la membrane (3), dans laquelle est disposée la pompe à vide (81).
